# EUROPÄISCHE PATENTSCHRIFT

(11) **EP 0 828 727 B1**
(45) Veröffentlichungstag und Bekanntmachung des Hinweises auf die Patenterteilung: **02.01.2003**
(21) Anmeldenummer: 96914207.4
(22) Anmeldetag: 14.05.1996
(51) Int. Cl.: C07D 307/94, C07D 307/86, A61K 31/34

(54) **CYCLOHEXYLDIHYDROBENZOFURANE**
CYCLOHEXYL DIHYDROBENZOFURANES
DIHYDROBENZOFURANES DE CYCLOHEXYLE

(30) Priorität: 18.05.1995 CH 147195; 22.01.1996 DE 19601911
(43) Veröffentlichungstag der Anmeldung: 18.03.1998
(73) Patentinhaber: ALTANA Pharma AG, 78467 Konstanz (DE)
(72) Erfinder: AMSCHLER, Hermann, xxxx (DE); BÄR, Thomas, D-78479 Reichenau (DE); FLOCKERZI, Dieter, D-78476 Allensbach (DE); SCHMID, Beate, D-78476 Allensbach (DE); THIBAUT, Ulrich, D-78464 Konstanz (DE); HATZELMANN, Armin, D-78467 Konstanz (DE); BOSS, Hildegard, D-78476 Allensbach (DE); HÄFNER, Dietrich, D-78464 Konstanz (DE); KLEY, Hans-Peter, D-78476 Allensbach (DE); BEUME, Rolf, D-78465 Konstanz (DE); ULRICH, Wolf-Rüdiger, D-78464 Konstanz (DE); MARTIN, Thomas, D-78462 Konstanz (DE)
(86) Internationale Anmeldenummer: EP9602055
(87) Internationale Veröffentlichungsnummer: WO96036626

(56) Entgegenhaltungen:
- WO-A-93/19747
- WO-A-93/19749
- WO-A-93/19751
- WO-A-94/02465

## Beschreibung

### Technisches Gebiet

Die Erfindung betrifft neue Verbindungen, die in der pharmazeutischen Industrie zur Herstellung von Medikamenten verwendet werden.

### Stand der Technik

In der internationalen Patentanmeldung WO92/12961 werden Benzamide mit PDE-hemmenden Eigenschaften beschrieben. In den internationalen Patentanmeldungen WO93/25517 und WO93/19749 werden trisubstituierte Phenylderivate als selektive PDE-IV-Hemmer offenbart. In der internationalen Patentanmeldung WO94/02465 werden Inhibitoren der c-AMP Phosphodiesterase und des TNF beschrieben.

### Beschreibung der Erfindung

Es wurde nun gefunden, daß die nachfolgend näher beschriebenen Dihydrobenzofurane, die sich von den vorveröffentlichten Verbindungen durch eine völlig andersartige Substitution unterscheiden, überraschende und besonders vorteilhafte Eigenschaften besitzen.

Gegenstand der Erfindung sind somit Verbindungen der Formel I (siehe beigefügtes Formelblatt I), worin
- R1: 1-6C-Alkoxy, 3-7C-Cycloalkoxy, 3-7C-Cycloalkylmethoxy, Benzyloxy oder ganz oder teilweise durch Fluor substituiertes 1-4C-Alkoxy bedeutet,
- R2: 1-4C-Alkyl und
- R3: Wasserstoff oder 1-4C-Alkyl bedeutet,
oder
- R2 und R3: gemeinsam und unter Einschluß der beiden Kohlenstoffatome, an die sie gebunden sind, einen 5-, 6- oder 7-gliedrigen, gewünschtenfalls durch ein Sauerstoffatom unterbrochenen Kohlenwasserstoffring darstellen,
- R4: Wasserstoff, Cyano, Carboxyl, 1-4C-Alkoxycarbonyl und Nitro und
- R5: Hydroxy, Carboxyl, 1-4C-Alkoxycarbonyl, Amino, Mono- oder Di-1-4C-alkylamino, 1-4C-Alkylcarbonylamino, 1-4C-Alkylsulfonylamino, Trifluormethylsulfonylamino oder Cyanamino, bedeutet,
und deren Salze.

1-6C-Alkoxy steht für einen Rest, der neben dem Sauerstoffatom einen geradkettigen oder verzweigten Alkylrest mit 1 bis 6 Kohlenstoffatomen enthält. Als Alkylreste mit 1 bis 6 Kohlenstoffatomen seien hierbei beispielsweise genannt der Hexyl-, Isohexyl- (2-Methylpentyl-), Neohexyl- (2,2-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Cycloalkoxy steht beispielsweise für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder teilweise durch Fluor substituiertes 1-4C-Alkoxy seien beispielsweise der 1,2,2-Trifluorethoxy-, der 2,2,3,3,3-Pentafluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

Als 5-, 6- oder 7-gliedriger, gewünschtenfalls durch ein Sauerstoffatom unterbrochener Kohlenwasserstoffring seien der Cyclopentan-, der Cyclohexan-, der Cycloheptan-, der Tetrahydrofuran- und der Tetrahydropyranring genannt. Wenn R2 und R3 gemeinsam und unter Einschluß der beiden Kohlenstoffatome, an die sie gebunden sind, einen 5-, 6- oder 7-gliedrigen Ring bilden, so liegt eine Spiroverbindung vor.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

1-4C-Alkoxycarbonyl steht für eine Carbonylgruppe, an die einer der vorstehend genannten 1-4C-Alkoxyreste gebunden ist. Beispielsweise seien der Methoxycarbonyl- (CH₃O-CO-) und der Ethoxycarbonylrest (CH₃CH₂O-CO-) genannt.

1-4C-Alkylcarbonyl steht für eine Carbonylgruppe, an die einer der vorstehend genannten 1-4C-Alkylreste gebunden ist. Beispielsweise sei der Acetylrest (CH₃CO-) genannt.

Als Mono- oder Di-1-4C-alkylaminoreste seien beispielsweise der Methylamino-, der Dimethylamino-, der Ethylamino-, der Diethylamino-, der Propylamino- und der Isopropylaminorest genannt.

Als 1-4C-Alkylcarbonylaminorest sei beispielsweise der Acetylaminorest (-NH-CO-CH₃) genannt.

1-4C-Alkylsulfonylamino steht für einen Sulfonylaminorest der durch einen der vorstehend genannten 1-4C-Alkylreste substituiert ist. Beispielsweise sei der Methylsulfonylaminorest (-NH-SO₂-CH₃) genannt.

Hervorzuhebende Verbindungen der Formel I sind solche, in denen
- R1: 1-4C-Alkoxy, 3-5C-Cycloalkoxy, 3-5C-Cycloalkylmethoxy oder ganz oder teilweise durch Fluor substituiertes 1-4C-Alkoxy bedeutet,
- R2: 1-4C-Alkyl und
- R3: Wasserstoff oder 1-4C-Alkyl bedeutet,
oder
- R2 und R3: gemeinsam und unter Einschluß der beiden Kohlenstoffatome, an die sie gebunden sind, einen Cyclopentan-, Cyclohexan-, Tetrahydrofuran- oder Tetrahydropyranring darstellen,
- R4: Wasserstoff, Cyano, Carboxyl oder 1-2C-Alkoxycarbonyl und
- R5: Hydroxy, Carboxyl, 1-2C-Alkoxycarbonyl, Amino oder Mono- oder Di-1-2C-alkylamino bedeutet,
und deren Salze.

Bevorzugte Verbindungen der Formel I sind solche, in denen
- R1: 1-4C-Alkoxy, 3-5C-Cycloalkoxy oder ganz oder teilweise durch Fluor substituiertes 1-4C-Alkoxy bedeutet,
- R2 und R3: gemeinsam und unter Einschluß der beiden Kohlenstoffatome, an die sie gebunden sind, einen Cyclopentan- oder Cyclohexanring darstellen,
- R4: Wasserstoff, Cyano, Carboxyl oder 1-2C-Alkoxycarbonyl und
- R5: Hydroxy, Carboxyl, 1-2C-Alkoxycarbonyl, Amino oder Mono- oder Di-1-2C-alkylamino bedeutet,
und deren Salze.

Besonders bevorzugte Verbindungen der Formel I sind solche, in denen
- R1: Methoxy,
- R2 und R3: gemeinsam und unter Einschluß der beiden Kohlenstoffatome, an die sie gebunden sind, einen Cyclopentanring darstellen,
- R4: Wasserstoff oder Cyano und
- R5: Hydroxy oder Carboxyl bedeutet,
und deren Salze.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren und Basen. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine einoder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Die Verbindungen der Formel I können als cis- oder trans-Isomere vorliegen und - sofern die Substitutionen -R2 und -CH₂R3 nicht identisch sind - handelt es sich auch um chirale Verbindungen. Die Erfindung umfaßt daher sowohl alle reinen Diastereomeren und reinen Enantiomeren als auch deren Gemische in jedem Mischungsverhältnis einschließlich der Racemate.

Die Enantiomeren können in an sich bekannter Weise (beispielsweise durch Herstellung und Trennung entsprechender diastereoisomerer Verbindungen) separiert werden.

Weiterer Gegenstand der Erfindung ist ein Verfahren zur Herstellung der Verbindungen der Formel I und ihrer Salze. Das Verfahren ist dadurch gekennzeichnet, daß man
a) Verbindungen der Formel II (siehe beigefügtes Formelblatt I), worin R1, R2, R3 und R4 die oben angegebenen Bedeutungen haben und Z ein Sauerstoffatom (O) bedeutet, unter die Carbonylgruppe gewünschtenfalls selektiv reduzierenden Bedingungen umsetzt oder daß man
b) zur Herstellung von Verbindungen der Formel I, worin R1, R2, R3 und R4 die oben angegebenen Bedeutungen haben und R5 eine Carboxylgruppe bedeutet, entsprechende Verbindungen der Formel II, worin Z die Gruppe C(Br)₂ bedeutet, hydrolysiert und daß man
gewünschtenfalls anschließend erhaltene Verbindungen der Formel I in ihre Salze überführt, oder daß man erhaltene Salze der Verbindungen der Formel I in die freien Verbindungen überführt.

Gewünschtenfalls können auch weitere Verbindungen der Formel I in einer dem Fachmann bekannten Weise durch Derivatisierung (insbesondere der Reste R4 und R5) in andere Verbindungen der Formel I übergeführt werden.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die Reduktion der Carbonylgruppe in Verbindungen der Formel II nach Variante a) wird in einer dem Fachmann bekannten Weise, vorzugsweise in geeigneten inerten Lösungsmitteln wie 1,2-Dimethoxyethan oder einem Alkohol wie Methanol, mit einem geeigneten Reduktionsmittel, wie beispielsweise Natriumborhydrid oder Lithiumborhydrid durchgeführt.

Die Hydrolyse von Verbindungen der Formel II nach Variante b), worin Z die Gruppe C(Br)₂ bedeutet, erfolgt durch Anwendung dem Fachmann bekannter Methoden, z.B. so wie in den nachfolgenden Beispielen beschrieben.

Verbindungen der Formel II, worin R1, R2 und R3 die oben genannten Bedeutungen haben, R4 Wasserstoff und Z Sauerstoff (0) bedeutet, können durch selektive Hydrierung der Kohlenstoffdoppelbindung im Cyclohexenring entsprechender Verbindungen der Formel III (siehe beigefügtes Formelblatt I) hergestellt werden.

Verbindungen der Formel II, worin R1, R2, R3 und R4 die obengenannten Bedeutungen haben und Z die Gruppe C(Br)₂ darstellt erhält man beispielsweise durch Umsetzung von entsprechenden Verbindungen der Formel II, worin Z Sauerstoff (O) bedeutet, mit Tetrabromkohlenstoff und Triphenylphosphan in einer dem Fachmann bekannten Weise.

Verbindungen der Formel II, worin R1, R2 und R3 die oben genannten Bedeutungen haben, R4 Cyano und Z Sauerstoff (O) bedeutet, können durch Anwendung dem Fachmann bekannter Methoden ausgehend von entsprechenden Verbindungen der Formel IV (siehe beigefügtes Formelblatt I), worin X die Gruppe -C(O)-OCH₃ bedeutet, gemäß dem allgemeinen Reaktionsschema auf dem beigefügten Formelblatt II hergestellt werden. Die Reaktionssequenz ist beispielhaft unter "Ausgangsverbindungen" beschrieben, die Herstellung weiterer Verbindungen kann analog erfolgen.

Verbindungen der Formel III, worin R1, R2 und R3 die oben genannten Bedeutungen haben, sind beispielsweise durch Addition entsprechender Verbindungen der Formel IV, worin X die Bedeutung Lithium hat, an 1,4-Cyclohexandion und anschließende Eliminierung von Wasser zugänglich. Zweckmäßigerweise wird das 1,4-Cyclohexandion in teilweise geschützter Form, beispielsweise als Monoethylenketal, eingesetzt und die Schutzgruppe nach erfolgter Umsetzung wieder abgespalten.

Verbindungen der Formel IV worin X Lithium bedeutet, sind aus entsprechenden Verbindungen der Formel IV worin X Halogen, insbesondere Brom, bedeutet durch Metall-Halogen-Austausch zugänglich.

Die Verbindungen der Formel IV worin R1, R2 und R3 die oben genannten Bedeutungen haben und X Halogen oder die Gruppe -C(O)-OCH₃ bedeutet, können gemäß dem allgemeinen Reaktionsschema auf dem beigefügten Formelblatt IIa hergestellt werden. Die Synthese von Verbindungen der Formel IV ist beispielhaft unter "Ausgangsverbindungen" beschrieben. Weitere Verbindungen der Formel IV können in analoger Weise hergestellt werden.

Die folgenden Beispiele erläutern die Erfindung näher, ohne sie einzuschränken. Die Abkürzung h steht für Stunde(n), Min. für Minute(n), RT für Raumtemperatur, Schmp. für Schmelzpunkt, DMSO für Dimethylsulfoxid, DMF für Dimethylformamid, THF für Tetrahydrofuran, Pd(DIPHOS)₂ für Bis-[bis-(1,2-diphenylphosphino)-ethan]-palladium(O), PEG-400 für Polyethylenglykol 400 und DC für Dünnschichtchromatographie.

### Beispiele

### Endprodukte

### 1. Cis- und Trans-4-Cyano-4-(2,3-Dihydro-7-methoxybenzofuran-2-spiro-1'cyclopentan-4-yl)-1-cyclohexanol

### 1.1 Cis-4-Cyano-4-(2,3-Dihydro-7-methoxybenzofuran-2-spiro-1'-cyclopentan-4-yl)-1-cyclohexanol

Natriumborhydrid (2,0 g, 52,4 mmol) wird unter Rühren bei RT in absolutes Methanol (70 ml) eingetragen. Anschließend wird eine Suspension von der nach A1 hergestellten Verbindung (6,3 g, 19,4 mmol) in absolutem Methanol (130 ml) zugetropft. Nach erneuter Zugabe von Natriumborhydrid (1,4 g, 36,7 mmol) wird das Reaktionsgemisch so lange mit 2N Salzsäure versetzt, bis ein pH-Wert von 2 erreicht ist. Zur Reaktionslösung gibt man eine gesättigte Natriumchloridlösung (300 ml) und destilliertes Wasser (100 ml) und extrahiert mit Essigsäureethylester (100 ml, 3mal). Die vereinigten organischen Phasen werden über Magnesiumsulfat getrocknet und nach Filtration im Vakuum eingeengt. Nach Flash-Chromatographie über Kieselgel [Petrolether/Essigsäureethylester (6:4)] wird die Titelverbindung (2,8 g) als farbloser Feststoff erhalten. [DC, Petrolether/Essigsäureethylester (6:4), Rf=0,29], Schmp. 143°C.

### 1.2 Trans-4-Cyano-4-(2,3-Dihydro-7-methoxybenzofuran-2-spiro-1'-cyclopentan-4-yl)-1-cyclohexanol

Die Trans-Hydroxy-Verbindung wurde aus dem gleichen Ansatz als farbloser Feststoff (0,75 g) erhalten. [DC, Petrolether/Essigsäureethylester (6:4), Rf=0,37], Schmp. 145°C.

### 2. Cis-4-(2,3-Dihydro-7-methoxybenzofuran-2-spiro-1'-cyclopentan-4-yl)-1-cyclohexanol

Zu einer Lösung von 0,11 g (0,36 mmol) der nach B1 hergestellten Verbindung in 10 ml 1,2-Dimethoxyethan gibt man 0,05 g (1,3 mmol) Natriumborhydrid und rührt für 12 h bei RT. Anschließend wird die Mischung mit 5 ml 2N Salzsäure, 50 ml destilliertem Wasser und 100 ml Ethylether versetzt, von der organischen Phase abgetrennt, mit Essigsäureethylester die wäßrige Phase nachextrahiert, die organischen Phasen über Natriumsulfat getrocknet und am Rotationsverdampfer eingedampft. Säulenchromatographie ergibt 47 mg der Titelverbindung als zähes Wachs. [DC (Nanoplatten, Petrolether/Essigsäureethylester, 6:4), Rf=0,23]

### 3. Cis-4-(2,3-Dihydro-7-methoxybenzofuran-2-spiro-1'-cyclopentan-4-yl)-1-cyclohexancarbonsäure

10 ml 10N Natronlauge werden zu 10 ml PEG-400 zugegeben und unter Stickstoff bei RT 1/2 h gerührt. Nach Zugabe von 0,1 g Pd(DIPHOS)₂ in 3 ml absolutem Methylenchlorid und 3 ml PEG-400 rührt man weitere 2 h bei RT unter Stickstoff, wobei sich die Lösung schwarz färbt. Anschließend tropft man eine Lösung von 1,1 g (2,4 mmol) der nach C1 hergestellten Verbindung in 5 ml absolutem Methylenchlorid und 5 ml PEG-400 zu und rührt bei 60°C für 20 h. Nach Abkühlen der Reaktionslösung säuert man mit 6N Salzsäure auf pH=2 an, trennt von der organischen Phase ab und schüttelt die wäßrige Phase nochmals mit 2x100 ml Essigsäureethylester aus. Die organischen Phasen werden vereinigt, über Natriumsulfat getrocknet, zur Trockne eingedampft und durch Säulenchromatographie sowie durch anschließendes Umkristallisieren aus Ether gereinigt. Man erhält 0,31 g der Titelverbindung als weißen Feststoff vom Schmp. 103-105°C.

### Ausgangsverbindungen

### A1. 4-Cyano-4-(2,3-dihydro-7-methoxybenzofuran-2-spiro-1'-cyclopentan-4-yl)cyclohexanon

0,6 g (1,56 mmol) der nach A2 hergestellten Verbindung löst man in einer Mischung von 1 ml DMSO, 1 ml destilliertem Wasser und 0,5 g Natriumchlorid und rührt bei 180-185° für 10 h. Nach Abkühlen der Mischung gibt man zu der Reaktionslösung 200 ml destilliertes Wasser und extrahiert mit Essigsäureethylester, trennt die organische Phase ab, trocknet über Natriumsulfat und dampft zur Trockne ein. Nach Flash-Chromatographie ergibt sich 0,4 g der Titelverbindung als gelblicher Feststoff vom Schmp. 92-94°C.

### A2. 5-Cyano-5-(2,3-dihydro-7-methoxy-benzofuran-2-spiro-1'-cyclopentan-4-yl)-2-oxocyclohexancarbonsäuremethylester

Zu einer Lösung von 1,2 g (0,29 mmol) der nach A3 hergestellten Verbindung in 25 ml 1,2-Dimethoxyethan gibt man unter Stickstoff bei RT portionsweise 0,25 g (0,82 mmol) Natriumhydrid (80 %ig in Paraffin), kocht für 6 h am Rückfluß, läßt anschließend auf RT abkühlen und rührt für weitere 48 h bei RT. Anschließend gibt man 5 ml Methanol und 10 ml 1N Salzsäure zu, versetzt danach mit 100 ml destilliertem Wasser und extrahiert mit Essigsäureethylester. Die organischen Phasen werden vereinigt, über Natriumsulfat getrocknet und zur Trockne eingedampft. Durch Säulenchromatographie erhält man 0,6 g der Titelverbindung als weißen Feststoff vom Schmp. 137-139°C.

### A3. 4-Cyano-4-(2,3-dihydro-7-methoxybenzofuran-2-spiro-1'-cyclopentan-4-yl)heptan-1,7-dicarbonsäuremethylester

Eine Lösung von 1,5 g (0,67 mmol) der nach A4 hergestellten Verbindung und 1 ml Triton B in 50 ml absolutem Acetonitril wird für 10 Min. auf 60°C erwärmt und anschließend werden bei dieser Temperatur 6 ml (0,66 mmol) Acrylsäuremethylester zugetropft. Die Lösung wird für 6 h am Rückfluß erhitzt. Nach dem Abkühlen wird das Lösemittel am Rotationsverdampfer abgezogen, der Rückstand in 150 ml Ethylether aufgenommen und mit halbgesättigter Natriumchloridlösung ausgeschüttelt. Die organische Phase wird getrocknet und am Rotationsverdampfer abgezogen. Säulenchromatographie ergibt 2,0 g der Titelverbindung als gelbes Öl. [DC (Petrolether/Essigsäureethylester 6:4) Rf=0,55].

### A4. 2,3-Dihydro-7-methoxybenzofuran-2-spiro-1'-cyclopentan-4-yl-acetonitril

2,4 g (0,01 mol) der nach A5 hergestellten Verbindung werden in 50 ml absolutem Toluol gelöst und unter Rühren mit 1 ml frischem Thionylchlorid versetzt. Die Mischung wird 1 h am Rückfluß gekocht. Nach Abkühlen auf RT zieht man das Lösemittel ab. Anschließend wird der Rückstand noch 3 mal zusammen mit jeweils 50 ml Toluol koevaporiert. Der Rückstand wird in 30 ml absolutem Toluol aufgenommen und zu einer Suspension von 0,5 g (0,01 mol) Natriumcyanid in 20 ml absolutem DMF und 1 ml 15-Crown-6 langsam bei RT zugetropft. Die Mischung wird für 4 h bei RT gerührt und anschließend auf 200 ml halbgesättigte Natriumchloridlösung gegeben und mit 200 ml Essigsäureethylester extrahiert. Die organischen Phasen werden vereinigt und über Natriumsulfat getrocknet. Man erhält nach Flash-Chromatographie 1,9 g der Titelverbindung als braunes Öl. [DC (Petrolether/Essigsäureethylester 6:4) Rf=0,83].

### A5. (2,3-Dihydro-7-methoxybenzofuran-2-spiro-1'-cyclopentan-4-yl)methanol

1,8 ml (6,0 mmol) Natriumdihydrido-bis(2-methoxyethoxy)aluminat (SDMA) werden unter Stickstoff zu 15 ml Toluol gegeben. 1,6 g (6,0 mmol) der nach A6 erhaltenen Verbindung werden in 10 ml Toluol gelöst und langsam unter ständigem Stickstoffstrom zu der SDMA-Lösung zugetropft. Nachdem alles zugetropft ist, rührt man noch für 30 Min. bei RT weiter und zieht anschließend das Lösemittel am Rotationsverdampfer ab. Zum Rückstand gibt man 100 ml destilliertes Wasser und extrahiert mit 200 ml Essigsäureethylester. Die organischen Phasen werden vereinigt, getrocknet und eingedampft. Man erhält 1,2 g eines hellgelben Öls, das durch Zugabe von n-Pentan spontan anfängt zu kristallisieren. [DC (Petrolether/Essigsäureethylester, 6:4) Rf=0,34].

### A6. 2,3-Dihydro-7-methoxybenzofuran-2-spiro-1'-cyclopentan-4-carbonsäuremethylester

10,2 g der nach A7 hergestellten Verbindung werden in 500 ml wasserfreiem n-Hexan gelöst und mit ca. 5 g Amberlyst 15 versetzt. Das Gemisch wird 3 Tage bei RT gerührt, filtriert und eingeengt. Der Rückstand wird über eine Kieselgelsäule mit Essigsäureethylester/Petrolether (4:6) chromatographiert, die chromatographisch reinen Fraktionen werden vereinigt, eingeengt und im Hochvakuum getrocknet. Man erhält 7,2 g der Titelverbindung als gelbes Öl.

### A7. 2-Cyclopenten-1-ylmethyl-3-hydroxy-4-methoxybenzoesäuremethylester

12,7 g der nach A8 hergestellten Verbindung werden mit 50 ml Chinolin versetzt und das Gemisch 1 h bei 190°C gerührt. Nach Abkühlen wird mit Wasser versetzt, mit 2N Salzsäure pH 3 eingestellt und mit Essigsäureethylester extrahiert. Der nach Einengen des Lösungsmittels verbleibende Rückstand wird über eine Kieselgelsäule mit Essigsäureethylester/Petrolether (4:6) chromatographiert. Die chromatographisch reinen Fraktionen werden eingeengt und im Hochvakuum getrocknet. Man erhält 10,2 g der Titelverbindung als gelbes Öl.

### A8. 3-(2-Methylencvclopentyloxy)-4-methoxybenzoesäuremethylester

28,5 g Methyltriphenylphosphoniumbromid werden in 300 ml wasserfreiem THF unter Stickstoff suspendiert und das Gemisch auf -40°C abgekühlt. Dann werden unter Rühren 50 ml n-Butyllithium (1,6 mol) in n-Hexan zugetropft. Nach 30 Min. Rühren bei -20 bis -10°C wird eine Lösung von 20 g der nach A9 hergestellten Verbindung in 100 ml abs. THF zugetropft. Danach läßt man das Gemisch sich auf RT erwärmen und rührt noch 1 h. Es wird auf Wasser gegossen und mit Essigsäureethylester extrahiert. Das nach Einengen der organischen Phase verbleibende Öl wird über eine Kieselgelsäule mit Essigsäureethylester/Petrolether (4:6) chromatographiert. Die chromatographisch reinen Fraktionen werden vereinigt, eingeengt und im Hochvakuum getrocknet. Man erhält 12,7 g der Titelverbindung als farbloses Öl.

### A9. 4-Methoxy-3-(2-oxocyclopentyloxy)benzoesäuremethylester

23,8 g 3-Hydroxy-4-methoxybenzoesäuremethylester werden in 200 ml wasserfreiem DMF gelöst und die Lösung mit 35 g Kaliumcarbonat (gemahlen) und 13 ml 2-Chlorcyclopentanon versetzt. Das Gemisch wird 3 h bei 60°C gerührt, dann vom Feststoff abgesaugt und das Filtrat im Vakuum eingeengt. Der Rückstand wird über eine Kieselgelsäule mit Essigsäureethylester/Petrolether (4:6) chromatographiert. Die chromatographisch reinen Fraktionen werden vereinigt, eingeengt und im Hochvakuum getrocknet. Man erhält 24,3 g der Titelverbindung als hellgelbes Öl.

### B1. 4-(2,3-Dihydro-7-methoxybenzofuran-2-spiro-1'-cyclopentan-4-yl)cyclohexanon

4,0 g (0,013 mol) der nach B2 erhaltenen Verbindung werden in 300 ml Ethanol gelöst, mit 0,3 g 10 %igem Pd/C versetzt und in einer Umlaufhydrierapparatur für 1 h hydriert. Nach Abfiltrieren und Nachwaschen des Katalysators mit Methanol wird die organische Phase am Rotationsverdampfer abgezogen und der Rückstand über Säulenchromatographie gereinigt. Man erhält 3,8 g der Titelverbindung als klares Öl [DC (Nanoplatten, Petrolether/Essigsäureethylester, 6:4) Rf=0,43].

### B2. 4-(2,3-Dihydro-7-methoxybenzofuran-2-spiro-1'-cyclopentan-4-yl)cyclohex-3-en-1-on

Zu einer Lösung von 7,6 g (0,021 mol) der nach B3 hergestellten Verbindung in 100 ml Toluol gibt man 50 ml destilliertes Wasser und 2 Spatelspitzen p-Toluolsulfonsäuremonohydrat und erhitzt für 2 h am Rückfluß. Nach Abkühlen wird die organische Phase am Rotationsverdampfer abgezogen, der Rückstand in 100 ml Aceton aufgenommen und eine Spatelspitze p-Toluolsulfonsäure zugesetzt. Nachdem die Mischung 8 h am Rückfluß gekocht hat, läßt man sie abkühlen, zieht Aceton ab, nimmt den Rückstand in Ether auf, versetzt mit 100 ml 1N Natronlauge, trennt die organische Phase ab und extrahiert mit Ether. Nach Vereinigen und Trocknen der organischen Phasen über Natriumsulfat wird eingeengt und das Produkt über Säulenchromatographie gereinigt. Man erhält 5,4 g der Titelverbindung als gelbes Öl. [DC (Nanoplatten, Petrolether/Essigsäureethylester, 6:4) Rf=0,41].

### B3. 4-Hydroxy-4-(2,3-dihydro-7-methoxybenzofuran-2-spiro-1'-cyclopentan-4-yl)cyclohexanonmonoethylenketal

Zu einer Lösung von 9,5 g (0,034 mol) der nach B4 hergestellten Verbindung in 100 ml absolutem THF gibt man bei -89°C tropfenweise unter Stickstoff 21,2 ml (0,036 mol) n-Butyllithium und rührt bei dieser Temperatur noch 30 Min. weiter. Anschließend tropft man eine Lösung von 5,3 g (0,34 mol) Cyclohexandionmonoethylenketal bei -78°C zu und rührt anschließend noch 2 h bei -70°C. Nach Erwärmen auf RT versetzt man mit 100 ml destilliertem Wasser und neutralisiert mit 1N Salzsäure. Es wird von der organischen Phase abgetrennt und die wäßrige Phase mit Methylenchlorid zusätzlich extrahiert. Die organischen Phasen werden vereinigt und über Natriumsulfat getrocknet, am Rotationsverdampfer eingedampft und über Säulenchromatographie gereinigt. Man erhält 7,1 g der Titelverbindung als gelbes Öl. [DC (Petrolether/Essigsäureethylester, 6:4) Rf=0,16].

### B4. 4-Brom-2,3-dihydro-7-methoxybenzofuran-2-spiro-1'-cyclopentan

Zu einer Lösung von 8,4 g (0,03 mol) der nach B5 hergestellten Verbindung in 100 ml absolutem Toluol gibt man 9,0 g Amberlist 15 und rührt die Mischung für 10 h bei 100°C. Nach Abkühlen der Mischung wird vom H+-Ionenaustauscher abfiltriert und mit 100 ml Methanol nachgewaschen. Nach Abziehen der organischen Phase und Säulenchromatographie erhält man 7,4 g der Titelverbindung als gelbes Öl [DC (Petrolether/Essigsäureethylester, 6:4) Rf=0,72].

### B5. 2-Cyclopent-1-enylmethyl-3-hydroxy-4-methoxybrombenzol

Zu einer Suspension von 26,5 g (0,074 mol) Methyltriphenylphosphoniumbromid in 200 ml absolutem THF gibt man bei -89°C unter Stickstoff tropfenweise 52,1 ml (0,082 mol) n-Butyllithium. Anschließend wird die Suspension auf -30°C erwärmt, wobei die Suspension in Lösung geht. Nach erneutem Abkühlen auf -70°C tropft man eine Lösung von 19,2 g (0,067 mol) der nach B6 hergestellten Verbindung in 200 ml absolutem THF unter Stickstoff zu. Anschließend wird auf -10°C erwärmt und 5 h bei dieser Temperatur gerührt. [DC (Petrolether/Essigsäureethylester, 6:4) Rf (Methylenverbindung) 0,81]. Nach Aufwärmen auf RT wird die Mischung von Feststoffen abfiltriert, das Filtrat mit 3x200 ml halbgesättigter Natriumchloridlösung und 2x200 ml destilliertem Wasser ausgeschüttelt. Nach Vereinigen der organischen Phasen, Trocknen über Natriumsulfat und Eindampfen bis zur Trockne wird der Rückstand in 50 ml Chinolin aufgenommen und 1 h bei 195-205°C gerührt. Nach Abkühlen der Lösung gibt man 400 ml Essigsäureethylester zu und schüttelt das Chinolin mit 4x200 ml 2N Salzsäure aus. Die organischen Phasen werden vereinigt, über Natriumsulfat getrocknet und am Rotationsverdampfer zur Trockne gebracht. Nach Säulenchromatographie ergibt sich eine Ausbeute von 8,4 g der Titelverbindung als rotbraunes Öl. [DC (Petrolether/Essigsäureethylester, 6:4) Rf=0,65].

### B6. 4-Methoxy-3-(2-oxocyclopentyloxy)brombenzol

Zu einer Lösung von 20 g (0,1 mol) der nach B7 hergestellten Verbindung in 300 ml absolutem DMF gibt man 17,7 g (0,15 mol) 2-Chlorcyclopentanon und 41,4 g (0,3 ml) Kaliumcarbonat und rührt 12 h bei RT. Nach Abfiltrieren der Feststoffe wird das Filtrat eingeengt, in 500 ml Essigsäureethylester aufgenommen und mit 3x200 ml destilliertem Wasser ausgeschüttelt. Säulenchromatographie ergibt 21,1 g der Titelverbindung als braunes Öl. [DC (Petrolether/Essigsäureethylester, 6:4) Rf=0,47].

### B7. 3-Hydroxy-4-methoxybrombenzol

Zu einer auf 8°C gekühlten Suspension von 50,0 g (0,23 mol) 5-Brom-2-methoxybenzaldehyd in 120 ml Methylenchlorid tropft man über einen Zeitraum von 1 h eine Suspension von 80 g (0,325 mol) m-Chlorperbenzoesäure in 500 ml absolutem Methylenchlorid zu, entfernt anschließend die Eiskühlung und läßt auf RT kommen. Die Mischung ist nach dem Zutropfen schneeweiß und verfärbt sich nach 70 h gelb. Durch DC-Kontrolle kann man die Umsetzung zum entsprechenden Ester verfolgen; DC (Petrolether/Essigsäureethylester, 6:4); Rf(5-Brom-2-methoxybenzaldehyd)=0,71; Rf(Ester) = 0,76. Nach 70 h Rühren bei RT ist die Umsetzung zum Ester laut DC quantitativ. Der Niederschlag wird von der Lösung abfiltriert, das Filtrat mit 5x200 ml 5 %iger Natriumsulfit-Lösung und 4x200 ml halbgesättigter Natriumhydrogencarbonatlösung gewaschen, anschließend mit 50 ml 2N Natronlauge versetzt und bei RT für 30 Min. stark gerührt. Man trennt die organische Phase von der basischen Phase ab und bringt die Natronlaugephase mit halbkonzentrierter Salzsäure auf pH 2. Nach Extrahieren mit 500 ml Essigsäureethylester und Trocknen der Essigsäureethylesterphase über Natriumsulfat wird die organische Phase am Rotationsverdampfer zur Trockne gebracht. Man erhält 45 g der Titelverbindung als dunkelrote Kristalle vom Schmp. 45-46°C.

### C1. 4-(2,3-Dihydro-7-methoxybenzofuran-2-spiro-1'-cyclopentan-4-yl)dibrommethylencyclohexan

Zu einer Lösung von 2,6 g (7,8 mmol) Tetrabromkohlenstoff in 20 ml absolutem Methylenchlorid gibt man unter Stickstoff 4,6 g (0,018 mol) Triphenylphosphan und rührt für 15 Min. bei RT wobei sich die Lösung orange färbt. Nach tropfenweiser Zugabe von 1,2 g (4 mmol) der nach B1 hergestellten Verbindung in 20 ml absolutem Methylenchlorid färbt sich die Lösung nach 1/2 h dunkelrot. Die organische Phase wird mit gesättigter Natriumchloridlösung gewaschen, über Natriumsulfat getrocknet und fast bis zur Trockne eingedampft. Zum Rückstand gibt man 15 g Kieselgel und 20 ml eines Petrolether/-Essigsäureethylester 8:2-Gemisches und dampft am Rotationsverdampfer bis zur Trockne ein. Säulenchromatographie ergibt 1,3 g der Titelverbindung als braunes Öl [DC (Petrolether/Essigsäureethylester, 8:2) Rf=0,74].

### Gewerbliche Anwendbarkeit

Die erfindungsgemäßen Verbindungen besitzen wertvolle pharmakologische Eigenschaften, die sie gewerblich verwertbar machen. Als selektive Zyklisch-Nukleotid Phosphodiesterase (PDE) Inhibitoren (und zwar des Typs IV) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigernden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des Darms, der Augen und der Gelenke, die vermittelt werden durch Mediatoren, wie Histamin, PAF (Plättchen- aktivierender Faktor), Arachidonsäure-Abkömmlinge wie Leukotriene und Prostaglandine, Zytokine, Interleukine, Chemokine, alpha-, beta- und gamma-Interferon, Tumornekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Harnleiter im Zusammenhang mit Nierensteinen; oder auch Erkrankungen des ZNS, wie beispielsweise Depressionen oder arteriosklerotische Demenz.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 0163965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 0,5 mg/kg. Die übliche Dosis bei systemischer Therapie liegt zwischen 0,05 und 2 mg/kg pro Tag.

### Biologische Untersuchungen

Bei der Untersuchung der PDE IV-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminol-verstärkte Chemolumineszenz gemessen werden kann. [Mc Phail LC, Strum SL, Leone PA und Sozzani S, The neutrophil respiratory burst mechanism. In "Immunology Series" 1992, 57, 47-76; ed. Coffey RG (Marcel Decker, Inc., New York-Basel-Hong Kong)].

Substanzen, welche die Chemolumineszenz sowie die Zytokinsekretion und die Sekretion entzündungssteigernder Mediatoren an Entzündungszellen, insbesonders neutrophilen und eosinophilen Granulozyten hemmen, sind solche, welche die PDE IV hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE IV-Hemmung durch die erfindungsgemäßen Substanzen ist damit ein zentraler Indikator für die Unterdrückung von entzündlichen Prozessen. (Giembycz MA, Could isoenzyme-selective phosphodiesterase inhibitors render bronchodilatory therapy redundant in the treatment of bronchial asthma?. Biochem Pharmacol 1992, 43, 2041-2051; Torphy TJ et al., Phosphodiesterase inhibitors: new opportunities for treatment of asthma. Thorax 1991, 46, 512-523; Schudt C et al., Zardaverine: a cyclic AMP PDE III/IV inhibitor. In "New Drugs for Asthma Therapy", 379-402, Birkhäuser Verlag Basel 1991; Schudt C et al., Influence of selective phosphodiesterase inhibitors on human neutrophil functions and levels of cAMP and Caᵢ. Naunyn-Schmiedebergs Arch Pharmacol 1991, 344, 682-690; Nielson CP et al., Effects of selective phosphodiesterase inhibitors on polymorphonuclear leukocyte respiratory burst. J Allergy Clin Immunol 1990, 86, 801-808; Schade et al., The specific type III and IV phosphodiesterase inhibitor zardaverine suppress formation of tumor necrosis factor by macrophages. European Journal of Pharmacology 1993, 230, 9-14).

### 1. Hemmung der PDE IV-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. 311, 193-198, 1980). Hierbei erfolgt im ersten Schritt die PDE-Reaktion. In einem zweiten Schritt wird das entstandene 5'-Nukleotid durch eine 5'-Nukleotidase des Schlangengiftes von ophiophagus hannah (King Cobra) zum ungeladenen Nukleosid gespalten. Im dritten Schritt wird das Nukleosid auf Ionenaustauschsäulen vom verbliebenen geladenen Substrat getrennt. Die Säulen werden mit 2 ml 30 mM Ammonium formiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte ergeben sich aus der folgenden Tabelle A, in der die Nummern der Verbindungen den Nummern der Beispiele entsprechen.

**Tabelle A**

| Hemmung der PDE IV-Aktivität | |
|---|---|
| Verbindung | -log IC₅₀ |
| 1 | 8,35 |
| 2 | 7,38 |
| 3 | 7,11 |

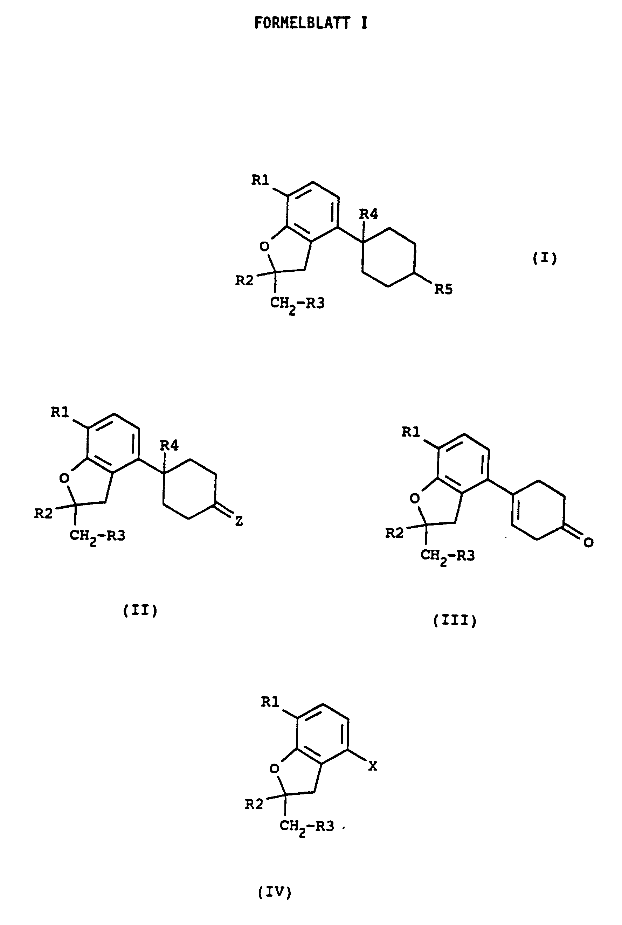

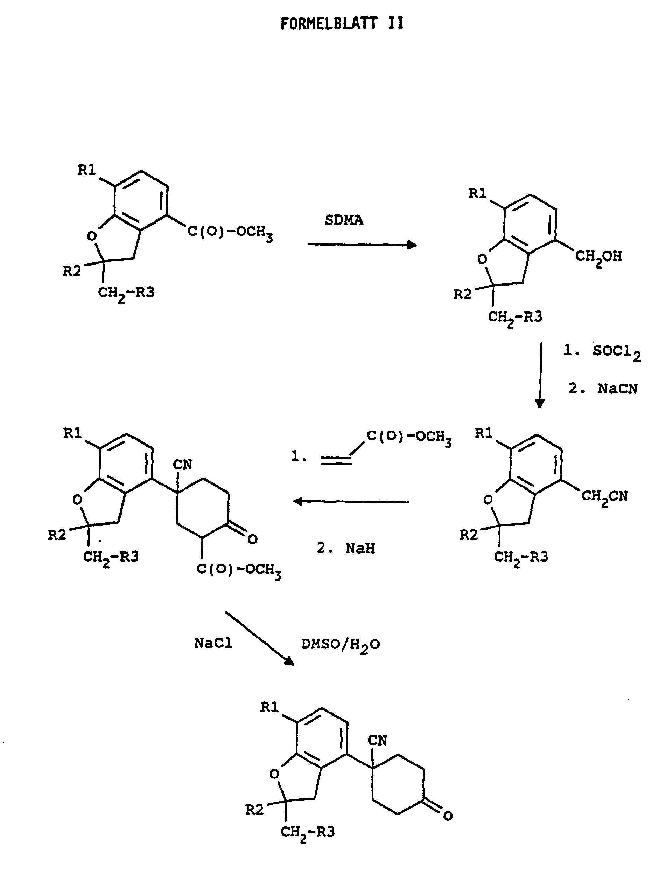

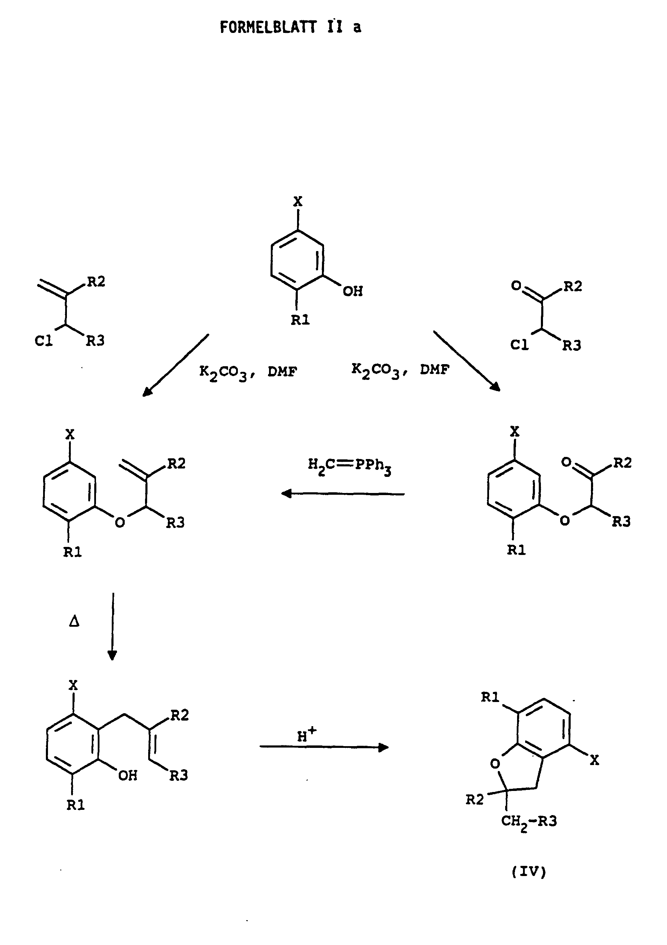

## Patentansprüche

1. Verbindungen der Formel I 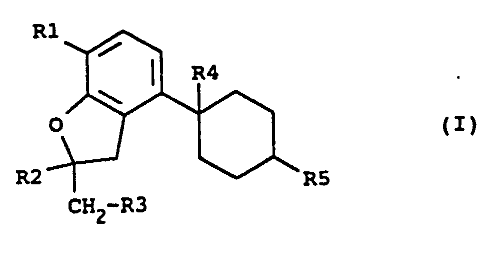 worin
R1 1-6C-Alkoxy, 3-7C-Cycloalkoxy, 3-7C-Cycloalkylmethoxy, Benzyloxy oder ganz oder teilweise durch Fluor substituiertes 1-4C-Alkoxy bedeutet,
R2 1-4C-Alkyl und
R3 Wasserstoff oder 1-4C-Alkyl bedeutet,
oder
R2 und R3 gemeinsam und unter Einschluß der beiden Kohlenstoffatome, an die sie gebunden sind, einen 5-, 6- oder 7-gliedrigen, gewünschtenfalls durch ein Sauerstoffatom unterbrochenen Kohlenwasserstoffring darstellen,
R4 Wasserstoff, Cyano, Carboxyl, 1-4C-Alkoxycarbonyl und Nitro und
R5 Hydroxy, Carboxyl, 1-4C-Alkoxycarbonyl, Amino, Mono- oder Di-1-4C-alkylamino, 1-4C-Alkylcarbonylamino, 1-4C-Alkylsulfonylamino, Trifluormethylsulfonylamino oder Cyanamino, bedeutet,
und deren Salze.

2. Verbindungen der Formel I nach Anspruch 1, in denen
R1 1-4C-Alkoxy, 3-5C-Cycloalkoxy, 3-5C-Cycloalkylmethoxy oder ganz oder teilweise durch Fluor substituiertes 1-4C-Alkoxy bedeutet,
R2 1-4C-Alkyl und
R3 Wasserstoff oder 1-4C-Alkyl bedeutet,
oder
R2 und R3 gemeinsam und unter Einschluß der beiden Kohlenstoffatome, an die sie gebunden sind, einen Cyclopentan-, Cyclohexan-, Tetrahydrofuran- oder Tetrahydropyranring darstellen,
R4 Wasserstoff, Cyano, Carboxyl oder 1-2C-Alkoxycarbonyl und
R5 Hydroxy, Carboxyl, 1-2C-Alkoxycarbonyl, Amino oder Mono- oder Di-1-2C-alkylamino bedeutet,
und deren Salze.

3. Verbindungen der Formel I nach Anspruch 1, in denen
R1 1-4C-Alkoxy, 3-5C-Cycloalkoxy oder ganz oder teilweise durch Fluor substituiertes 1-4C-Alkoxy bedeutet,
R2 und R3 gemeinsam und unter Einschluß der beiden Kohlenstoffatome, an die sie gebunden sind, einen Cyclopentan- oder Cyclohexanring darstellen,
R4 Wasserstoff, Cyano, Carboxyl oder 1-2C-Alkoxycarbonyl und
R5 Hydroxy, Carboxyl, 1-2C-Alkoxycarbonyl, Amino oder Mono- oder Di-1-2C-alkylamino bedeutet,
und deren Salze.

4. Verbindungen der Formel I nach Anspruch 1, in denen
R1 Methoxy,
R2 und R3 gemeinsam und unter Einschluß der beiden Kohlenstoffatome, an die sie gebunden sind, einen Cyclopentanring darstellen,
R4 Wasserstoff oder Cyano und
R5 Hydroxy oder Carboxyl bedeutet,
und deren Salze.

5. Verfahren zur Herstellung der Verbindungen der Formel I nach Anspruch 1 und ihrer Salze, **dadurch gekennzeichnet, daß** man
a) Verbindungen der Formel II, 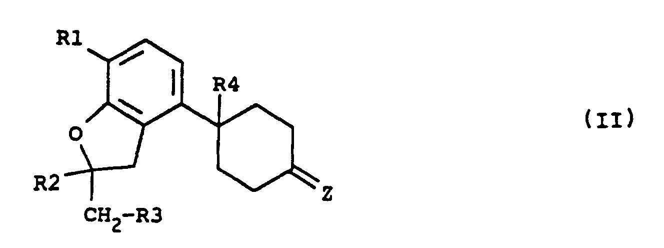 worin R1, R2, R3 und R4 die in Anspruch 1 angegebenen Bedeutungen haben und Z ein Sauerstoffatom (O) bedeutet, unter die Carbonylgruppe gewünschtenfalls selektiv reduzierenden Bedingungen umsetzt oder daß man
b) zur Herstellung von Verbindungen der Formel I, worin R1, R2, R3 und R4 die in Anspruch 1 angegebenen Bedeutungen haben und R5 eine Carboxylgruppe bedeutet, entsprechende Verbindungen der Formel II, worin Z die Gruppe C(Br)₂ bedeutet, hydrolysiert und daß man
gewünschtenfalls anschließend erhaltene Verbindungen der Formel I in ihre Salze überführt, oder daß man erhaltene Salze der Verbindungen der Formel I in die freien Verbindungen überführt.

6. Arzneimittel enthaltend eine oder mehrere Verbindungen nach Anspruch 1 zusammen mit üblichen pharmazeutischen Hilfs- und/oder Trägerstoffen.

7. Verbindung nach Anspruch 1 zur Anwendung bei der Behandlung von Krankheiten.

8. Verwendung von Verbindungen nach Anspruch 1 zur Herstellung von Arzneimitteln.

9. Verwendung von Verbindungen nach Anspruch 1 zur Herstellung von Arzneimitteln für die Behandlung von Atemwegserkrankungen.

10. Verwendung von Verbindungen nach Anspruch 1 zur Herstellung von Arzneimitteln für die Behandlung von Dermatosen.

## Claims

1. Compounds of the formula I 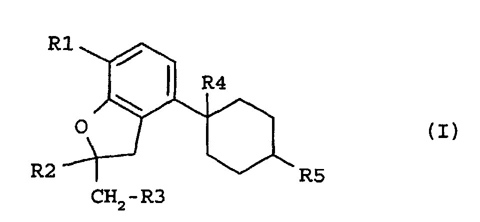 in which
R1 is 1-6C-alkoxy, 3-7C-cycloalkoxy, 3-7C-cydoalkylmethoxy, benzyloxy or 1-4C-alkoxy which is completely or partially substituted by fluorine,
R2 is 1-4C-alkyl and
R3 is hydrogen or 1-4C-alkyl,
or
R2 and R3, together and including the two carbon atoms to which they are bonded, are a 5-, 6- or 7-membered hydrocarbon ring, optionally interrupted by an oxygen atom,
R4 is hydrogen, cyano, carboxyl, 1-4C-alkoxycarbonyl and nitro and
R5 is hydroxyl, carboxyl, 1-4C-alkoxycarbonyl, amino, mono- or di-1-4C-alkylamino, 1-4C-alkylcarbonylamino, 1-4C-alkylsulphonylamino, trifluoromethylsulphonylarnino or cyanoamino,
and their salts.

2. Compounds of the formula I according to Claim 1, in which
R1 is 1-4C-alkoxy, 3-5C-cycloalkoxy, 3-5C-cycloalkylmethoxy or 1-4C-alkoxy which is completely or partially substituted by fluorine,
R2 is 1-4C-alkyl and
R3 is hydrogen or 1-4C-alkyl,
or
R2 and R3, together and including the two carbon atoms to which they are bonded, are a cyclopentane, cyclohexane, tetrahydrofuran or tetrahydropyran ring,
R4 is hydrogen, cyano, carboxyl or 1-2C-alkoxycarbonyl and
R5 is hydroxyl, carboxyl, 1-2C-alkoxycarbonyl, amino or mono- or di-1-2C-alkylamino,
and their salts.

3. Compounds of the formula I according to Claim 1, in which
R1 is 1-4C-alkoxy, 3-5C-cycloalkoxy or 1-4C-alkoxy which is completely or partially substituted by fluorine,
R2 and R3, together and including the two carbon atoms to which they are bonded, are a cyclopentane or cyclohexane ring,
R4 is hydrogen, cyano, carboxyl or 1-2C-alkoxycarbonyl and
R5 is hydroxyl, carboxyl, 1-2C-alkoxycarbonyl, amino or mono- or di-1-2C-alkylamino,
and their salts.

4. Compounds of the formula I according to Claim 1, in which
R1 is methoxy,
R2 and R3, together and including the two carbon atoms to which they are bonded, are a cyclopentane ring,
R4 is hydrogen or cyano and
R5 is hydroxyl or carboxyl,
and their salts.

5. Process for the preparation of the compounds of the formula I according to Claim 1 and their salts, **characterized in that**
a) compounds of the formula II 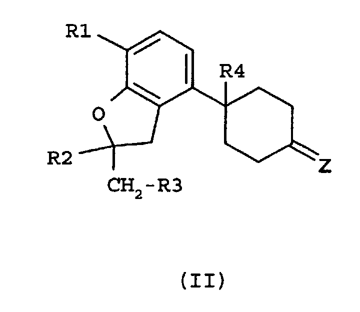 in which R1, R2, R3 and R4 have the meanings indicated in Claim 1 and Z is an oxygen atom (O) are reacted under conditions which, optionally, selectively reduce the carbonyl group or **in that**
b) for the preparation of the compounds of the formula I, in which R1, R2, R3 and R4 have the meanings indicated in Claim 1 and R5 is a carboxyl group, corresponding compounds of formula II, in which Z represents the group C(Br)₂ are hydrolysed and **in that**
optionally compounds of the formula I obtained are then converted into their salts, or **in that** salts of the compounds of the formula I obtained are converted into the free compounds.

6. Medicaments comprising one or more compounds according to Claim 1 together with customary pharmaceutical auxiliaries and/or excipients.

7. Compound according to Claim 1 for use in the treatment of illnesses.

8. Use of compounds according to Claim 1 for the production of medicaments.

9. Use of compounds according to Claim 1 for the production of medicaments for the treatment of airway disorders.

10. Use of compounds according to Claim 1 for the production of medicaments for the treatment of dermatoses.

## Revendications

1. Composés de formule I: 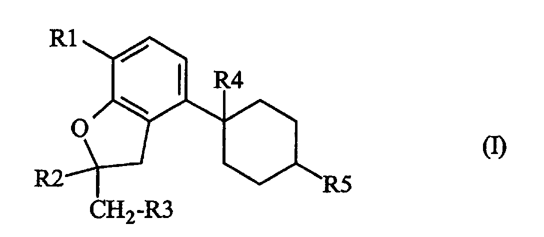 dans laquelle
R1 représente un groupe alcoxy en C₁ à C₆, un groupe cycloalcoxy en C₃ à C₇, un groupe cycloalkyl(C₃ à C₇)méthoxy, un groupe benzyloxy ou un groupe alcoxy en C₁ à C₄ totalement ou partiellement substitué par du fluor,
R2 représente un groupe alkyle en C₁ à C₄
et
R3 représente un atome d'hydrogène ou un groupe alkyle en C₁ à C₄,
ou bien
R2 et R3 forment ensemble et avec les deux atomes de carbone auxquels ils sont liés, un cycle hydrocarboné à 5, 6 ou 7 chaînons, éventuellement interrompu par un atome d'oxygène,
R4 représente un atome d'hydrogène ou un groupe cyano, carboxyle, alcoxy(C₁ à C₄)carbonyle ou nitro, et
R5 représente un groupe hydroxyle, carboxyle, alcoxy(C₁ à C₄)carbonyle, amino, monoalkyl(C₁ à C₄)amino, dlalkyl(C₁ à C₄)amino, alkyl(C₁ à C₄) carbonylamino, alkyl(C₁ à C₄)sulfonylamino, trifluorométhylsulfonylamino ou cyanamino,
et leurs sels.

2. Composés de formule (I) selon la revendication 1, pour lesquels :
R1 représente un groupe alcoxy en C₁ à C₄, un groupe cycloalcoxy en C₃ à C₅, un groupe cycloalkyl(C₃ à C₅)méthoxy ou un groupe alcoxy en C₁ à C₄ totalement ou partiellement substitué par du fluor,
R2 représente un groupe alkyle en C₁ à C₄ et
R3 représente un atome d'hydrogène ou un groupe alkyle en C₁ à C₄,
ou bien
R2 et R3 forment ensemble et avec les deux atomes de carbone auxquels ils sont liés, un cycle cyclopentane, cyclohexane, tétrahydrofurane ou tétrahydropyrane,
R4 représente un atome d'hydrogène ou un groupe cyano, carboxyle ou alcoxy(C₁ à C₂) carbonyle, et
R5 représente un groupe hydroxyle, carboxyle, alcoxy(C₁ à C₂) carbonyle, amino, monoalkyl(C₁ à C₂)amino ou dialkyl(C₁ à C₂)amino,
et leurs sels.

3. Composés de formule (I) selon la revendication 1, pour lesquels:
R1 représente un groupe alcoxy en C₁ à C₄, un groupe cycloalcoxy en C₃ à C₅ ou un groupe alcoxy en C₁ à C₄ totalement ou partiellement substitué par du fluor,
R2 et R3 forment ensemble et avec les deux atomes de carbone auxquels ils sont liés, un cycle cyclopentane ou cyclohexane,
R4 représente un atome d'hydrogène ou un groupe cyano, carboxyle ou alcoxy(C₁ à C₂)carbonyle, et
R5 représente un groupe hydroxyle, carboxyle, alcoxy(C₁ à C₂)carbonyle, amino, mono-alkyl(C₁ à C₂)amino ou dialkyl(C₁ à C₂)amino,
et leurs sels.

4. Composés de formule (I) selon la revendication 1, pour lesquels :
R1 représente un groupe méthoxy,
R2 et R3 forment ensemble et avec les deux atomes de carbone auxquels ils sont liés, un cycle cyclopentane,
R4 représente un atome d'hydrogène ou un groupe cyano et
R5 représente un groupe hydroxyle ou carboxyle,
et leurs sels.

5. Procédé de préparation des composés de formule (I) selon la revendication 1, et de leurs sels, **caractérisé en ce que** :
a) on fait réagir les composés de formule (II) 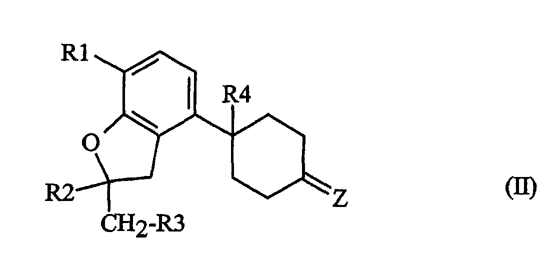 dans laquelle R1, R2, R3 et R4 ont les significations indiquées dans la revendication 1 et Z représente un atome d'oxygène, dans des conditions provoquant la réduction, sélective si on le souhaite, du groupe carbonyle, ou bien
b) pour la préparation des composés de formule (I) pour lesquels R1, R2, R3 et R4 ont les significations indiquées dans la revendication 1 et R5 représente un groupe carboxyle, on hydrolyse les composés correspondants de formule (II) pour lesquels Z représente un groupe C(Br)₂,
et ensuite on transforme éventuellement les composés obtenus de formule (I) en leurs sels ou les sels obtenus des composés de formule (I) en les composés libres.

6. Médicament contenant un ou plusieurs composés selon la revendication 1 avec des produits auxiliaires et/ou supports pharmaceutiques habituels.

7. Composé selon la revendication 1, qui est destiné à être utilisé pour le traitement de maladies.

8. Utilisation des composés selon la revendication 1 pour la préparation de médicaments.

9. Utilisation des composés selon la revendication 1, pour la préparation de médicaments destinés au traitement des affections des voies respiratoires.

10. Utilisation des composés selon la revendication 1 pour la préparation de médicaments destinés au traitement des dermatoses.
